# EUROPEAN PATENT SPECIFICATION

(11) **EP 3 574 854 B1**
(45) Date of publication and mention of the grant of the patent: **14.04.2021**
(21) Application number: 19177289.6
(22) Date of filing: 29.05.2019
(51) Int. Cl.: A61B 17/17, A61B 17/88, A61B 17/00, A61B 17/32

(54) **AN ALIGNMENT APPARATUS FOR GUIDING A CUTTING ELEMENT**
AUSRICHTVORRICHTUNG ZUM FÜHREN EINES SCHNEIDELEMENTS
APPAREIL D'ALIGNEMENT POUR GUIDER UN ÉLÉMENT DE DÉCOUPE

(30) Priority: 30.05.2018 NL 2021017
(43) Date of publication of application: 04.12.2019
(73) Proprietor: Temmerman, Olivier Paul Pieter, 2061 DE Bloemendaal (NL)
(72) Inventor: Temmerman, Olivier Paul Pieter, 2061 DE Bloemendaal (NL); Paalman, Micha Ilan, 1095 MA Amsterdam (NL); Klap, Paulien, 1018 DT Amsterdam (NL)
(74) Representative: Van Breda, Jacobus

(56) References cited:
- US-A1- 2005 203 508
- US-A1- 2006 149 269
- US-A1- 2009 216 232

## Description

The invention relates to an alignment apparatus for guiding a cutting element along a predetermined path in an intramedullary canal of a human bone, said apparatus comprising:
- a frame for guiding the cutting element along the predetermined path;
- an adjustable clamp assembly, connected to said frame, for clamping the frame to the bone.

Such an alignment apparatus for guiding a cutting element is known from US 5,312,409.

The known alignment apparatus of US 5,312,409 is used in hip replacement surgery, wherein a prosthesis is connected to the femur via cement or other adhesive material provided in the femoral canal. The known prior art acknowledges that when a previously inserted prosthesis must be replaced, it is necessary to remove at least a part of the original cement or adhesive from the femoral canal. When reaming out the old cement or adhesive, it is essential that no damage be done to the canal or femoral shaft, since perforations may prevent adequate fixation of the new prosthesis or weaken the femur. US 5,312,409 teaches to apply a frame for guiding the cutting element along a predetermined path, and an adjustable clamp assembly connected to the frame for clamping the outside of the bone such that the intramedullary canal of the clamp bone is disposed along the predetermined path.

It is an object of the invention to avoid the bulky construction of US 5,312,409, and to provide improved accuracy during removal of the cement from the femur bone, whilst also the danger of perforating the femur bone is minimized.

From US2005/0203508 an alignment apparatus according to the preamble of claim 1 is known, to note an alignment apparatus for guiding a cutting element along a predetermined path in an intramedullary canal of a human bone, said apparatus comprising:
- a frame for guiding the cutting element along the predetermined path;
- an adjustable clamp assembly, connected to said frame, for clamping the frame to the bone;
wherein:
- the frame comprises at least one tube with a lumen for guiding the cutting element, and which frame is arranged to be inserted into the said intramedullary canal; and
- the clamp assembly is provided at a distal end of the at least one tube;
- the frame comprises an inner tube and an outer tube that are coaxially positioned with respect to each other; which inner tube and outer tube are arranged to be inserted into said intramedullary canal;
- the inner tube is provided with the lumen for guiding the cutting element; and
- the clamp assembly is configured to be actuable for clamping to the bone by a relative motion of the inner tube and outer tube with respect to each other. This known device is used to facilitate alignment of instruments used to create a cavity in the femoral bone for reception of an femoral implant and to facilitate size selection of the femoral implant.

The invention proposes an alignment apparatus having the features of one or more of the appended claims.

In a first aspect of the invention the features of the preamble of claim 1 are completed with the features that:
- the clamp assembly is provided on the inner tube and comprises at least a first spreader which is embodied with two rings distant from each other that are mounted on and movable along the inner tube, and that are connected to each other with flexible legs;
   - a first stop is provided at a distal end of the inner tube to prevent that the first spreader slides off the inner tube;
   - a second stop is embodied by a distal end of the outer tube which limits movement of at least one of the two rings of the first spreader;
   - relative motion of the two rings of the first spreader causes flexing of the flexible legs, such that an approaching motion of the two rings of the first spreader causes the flexible legs to flex out and engage the intramedullary canal so as to act as a clamp within said intramedullary canal.

This enables a very secure method of removing the cement from the femur bone, which is both accurate and reliable and avoids the danger of perforating the femur bone. One favourable aspect of the alignment apparatus of the invention is that the cutting element that is used and that is guided by the alignment apparatus is very reliably positioned and maintained in position within the intramedullary canal of the femur bone, because of the fact that the clamp assembly is during operation positioned within said intramedullary canal close to the cement being cut.

Actuating the clamp assembly is easy and goes without sacrificing the benefits of the limited dimensions of the alignment apparatus of the invention due to the features that:
- the frame comprises an inner tube and an outer tube that are coaxially positioned with respect to each other; which inner tube and outer tube are arranged to be inserted into said intramedullary canal;
- the inner tube is provided with the lumen for guiding the cutting element; and
- the clamp assembly is provided on the inner tube and is configured to be actuable for clamping to the bone by a relative motion of the inner tube and outer tube with respect to each other.

It is preferred that the clamp assembly is actuable for clamping to the bone by a relative longitudinal motion of the inner tube and outer tube with respect to each other.

Favourable results may be achieved when:
- the clamp assembly comprises a second spreader, the first and second spreaders being embodied with two rings distant from each other that are mounted on and movable along the inner tube, and which rings of any such spreader are connected to each other with flexible legs;
- the first and second spreaders are provided adjacent to each other on the inner tube.

These two spreaders improve the stabilization of the cutting elements in the intramedullary canal by their joint action on the wall of said canal.

The object of the invention to stabilize the cutting element is further improved with the feature that the or each spreader is embodied with six legs. This provides an effective clamping in the intramedullary canal and leaves sufficient room within the legs that may be useful for removal of the cement that has been cut loose.

In one embodiment the rings of the spreaders that are facing each other are provided with cooperating protrusions and recesses to provide that the spreaders can be rotationally fixed with respect to each other. The legs of the respective spreaders can then be provided with different orientations.

Beneficially at a proximal end the apparatus, the same is provided with a handle and means to move the outer tube relative to the inner tube. There are different embodiments possible for the means to move the outer tube relative to the inner tube, and the following disclosure concerns a particular embodiment which may be preferred because of its ease of construction, although this embodiment must not be considered the only option.

In one suggested embodiment the means to move the inner tube relative to the outer tube is embodied as a trigger having at least one plate which is provided with an aperture for passing through the inner tube, and that operating the trigger initially causes the plate to tilt with reference to the inner tube and provide a connection to the inner tube, such that with continued movement of the trigger the inner tube moves longitudinally within the outer tube for actuating the clamp assembly.

In line with the previous paragraph it is possible that the proximal end of the apparatus is further provided with a release for the inner tube, which release is embodied with at least one plate provided with an aperture for passing through the inner tube, and which plate has a preferential position in which it is able to tilt relative to the inner tube and to connect to said inner tube, wherein operating the release causes the plate to be released and disconnected from the inner tube so as to enable the inner tube to move longitudinally within the outer tube to a release position of the clamp assembly which brings it in disengagement with the intramedullary canal of the bone.

The invention will hereinafter be further elucidated with reference to the drawing of an exemplary embodiment of an alignment apparatus according to the invention that is not limiting as to the appended claims.

In the drawing:
- figure 1 shows an alignment apparatus according to the invention in a side view with a phantom view at a part of a femur bone;
- figure 2 shows the alignment apparatus of figure 1 in an isometric and partly phantom view together with a bore instrument;
- figure 3 shows a spreader in a relaxed condition; and
- figure 4 shows a spreader in a flexed condition.

Whenever in the figures the same reference numerals are applied, these numerals refer to the same parts.

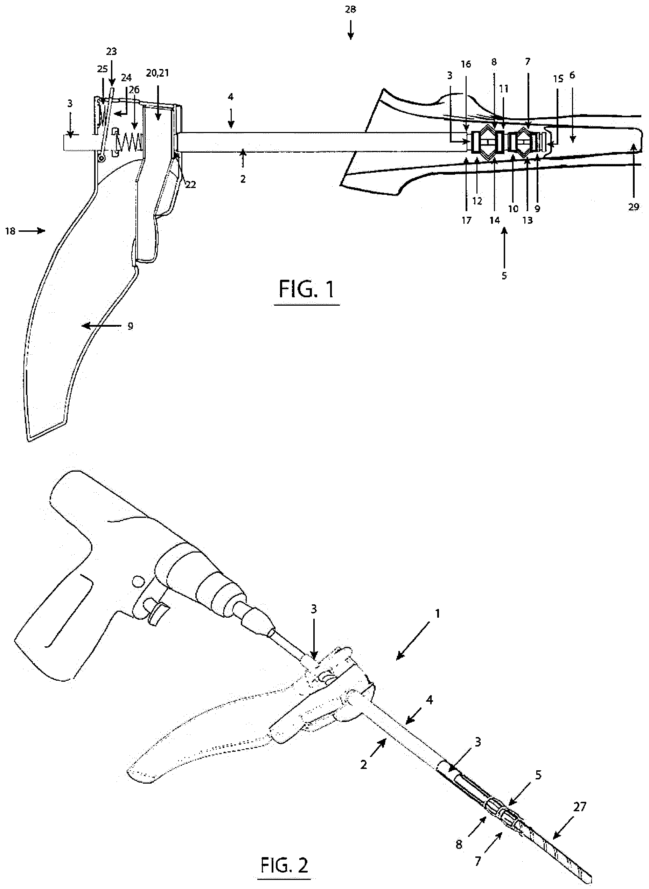

With reference to figure 1 and figure 2 an alignment apparatus 1 according to the invention is shown for guiding a cutting element 27 (in this example embodied as a drill; see figure 2) along a predetermined path in an intramedullary canal 29 of a human bone 28 (figure 1).

The alignment apparatus 1 of the invention comprises a frame 2 for guiding the cutting element 27 along the predetermined path which comprises at least one tube 3, 4 with a lumen for guiding the cutting element 27, and which frame 3, 4 is arranged to be inserted into the said intramedullary canal 29. The alignment apparatus 1 further comprises a clamp assembly 5 at a distal end 6 of the at least one tube 3, 4. This adjustable clamp assembly 5 is used for clamping the frame 2 comprising the at least one tube 3, 4 to the bone of the intramedullary canal 29.

In the shown embodiment the frame 2 comprises an inner tube 3 and an outer tube 4 that are coaxially positioned with respect to each other; and which inner tube 3 and outer tube 4 are arranged to be inserted into said intramedullary canal 29.

The inner tube 3 is provided with the lumen for guiding the cutting element 27 and the clamp assembly 5 is provided on the inner tube 3 and is actuable for clamping to the bone by a relative motion of the inner tube 3 and outer tube 4 with respect to each other, preferably by a relative longitudinal motion of the inner tube 3 and outer tube 4 with respect to each other.

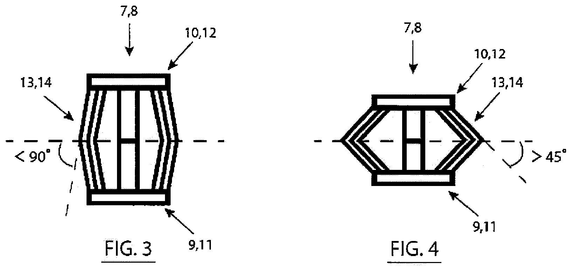

As both figure 1 and figure 2 show the clamp assembly 5 comprises at least one but preferably two spreaders 7, 8. A detail of one such spreader 7, 8 is shown in figure 3 in a relaxed condition and in figure 4 in a flexed condition. Each spreader 7, 8 is embodied with two rings 9, 10 and 11, 12 distant from each other that are mounted on and movable along the inner tube 3. The two rings 9, 10 and 11, 12 of each spreader 7, 8 are connected to each other with flexible legs 13, 14.

Further figure 1 shows that a first stop 15 is provided at a distal end 6 of the inner tube 3 to prevent that the spreader or spreaders 7, 8 slide off the inner tube 3; and a second stop 16 is embodied by a distal end 17 of the outer tube 4 which limits movement of the adjacent ring 12 of the spreader 8.

The disclosed construction provides that relative motion of the two rings 9, 10 of spreader 7 and relative motion of the two rings 11, 12 of spreader 8 causes flexing of the flexible legs 13 of spreader 7 and flexing of the flexible legs 14 of spreader 8 (as shown in figure 4), such that an approaching motion of the two rings of each spreader 7, 8 causes the flexible legs 13, 14 to flex out and engage the intramedullary canal 29 so as to act as a clamp within said intramedullary canal (see figure 1). The rings 10, 11 of the spreaders 7, 8 that are facing each other can be provided with cooperating protrusions and recesses to provide that the spreaders 7, 8 can be rotationally fixed with respect to each other. This is not shown in the figures but is clear to the artisan and requires no further elucidation. Preferably the or each spreader 7, 8 is embodied with six legs 13, 14.

Figure 1 and figure 2 further show that the alignment apparatus of the invention has at its proximal end 18 a handle 9 and means 20 to move the inner tube 3 relative to the outer tube 4. The means 20 to move the inner tube 3 relative to the outer tube 4 is embodied as a trigger 21 comprising at least one plate 22 which is provided with an aperture for passing through the inner tube 3, wherein operating the trigger 21 initially causes the plate 22 to tilt with reference to the inner tube 3 and provide a frictional connection to the inner tube 3, such that with continued movement of the trigger 21 the inner tube 3 moves longitudinally within the outer tube 4 for actuating the clamp assembly 5. Releasing the trigger 21 will return it to its preferential position as shown in figure 1 due to the action of a return spring 26.

Also at its proximal end 18 the apparatus 1 has a release 23 for the inner tube 3, which release 23 is embodied with at least one plate 24 provided with an aperture for passing through the inner tube 3, and which plate 24 has a preferential position in which it tilts relative to the inner tube 3 wherein it connects due to friction to said inner tube 3, and wherein operating the release 23 causes the plate 24 to be released and to be disconnected from the inner tube 3 so as to enable the inner tube 3 to move longitudinally within the outer tube 4 to a release position of the clamp assembly 5 which brings it in disengagement with the intramedullary canal 29 of the bone. The spreader or spreaders 7, 8 then assumed the position shown in figure 3. The alignment apparatus 1 can then be retracted from the intramedullary canal 29 into which it was inserted. Figure 1 also shows that the plate 24 of the release 23 is spring-loaded with a spring 25 which provides that the release 23 is brought back in a position wherein it tilts again with reference to the inner tube 3, when the release 23 is no longer actuated.

Although the invention has been discussed in the foregoing with reference to an exemplary embodiment of the alignment apparatus of the invention, the invention is not restricted to this particular embodiment which can be varied in many ways without departing from the invention. The discussed exemplary embodiment shall therefore not be used to construe the appended claims strictly in accordance therewith. On the contrary the embodiment is merely intended to explain the wording of the appended claims without intent to limit the claims to this exemplary embodiment. The scope of protection of the invention shall therefore be construed in accordance with the appended claims only, wherein a possible ambiguity in the wording of the claims shall be resolved using this exemplary embodiment.

## Claims

1. An alignment apparatus (1) for guiding a cutting element along a predetermined path in an intramedullary canal of a human bone, said apparatus comprising:
- a frame (2) for guiding the cutting element along the predetermined path;
- an adjustable clamp assembly (5), connected to said frame (2), for clamping the frame (2) to the bone;
wherein:
- the frame (2) comprises at least one tube (3, 4) with a lumen for guiding the cutting element, and which frame (2) is arranged to be inserted into the said intramedullary canal; and
- the clamp assembly (5) is provided at a distal end (6) of the at least one tube (3, 4);
- the frame (2) comprises an inner tube (3) and an outer tube (4) that are coaxially positioned with respect to each other; which inner tube (3) and outer tube (4) are arranged to be inserted into said intramedullary canal;
- the inner tube (3) is provided with the lumen for guiding the cutting element;
- the clamp assembly (5) is configured to be actuable for clamping to the bone by a relative motion of the inner tube (3) and outer tube (4) with respect to each other;
**characterized in that**
- the clamp assembly (5) is provided on the inner tube (3) and comprises at least a first spreader (7, 8) which is embodied with two rings (9, 10; 11, 12) distant from each other that are mounted on and movable along the inner tube (3), and that are connected to each other with flexible legs (13, 14);
- a first stop (15) is provided at a distal end (6) of the inner tube (3) to prevent that the first spreader (7, 8) slides off the inner tube (3);
- a second stop (16) is embodied by a distal end (17) of the outer tube (4) which limits movement of at least one (12) of the two rings of the first spreader (7, 8);
- relative motion of the two rings (9, 10; 11, 12) of the first spreader (7, 8) will cause flexing of the flexible legs (13, 14), such that an approaching motion of the two rings (9, 10; 11, 12) of the first spreader (7, 8) will cause the flexible legs (13, 14) to flex out and engage the intramedullary canal so as to act as a clamp within said intramedullary canal.

2. Alignment apparatus according to claim 1, **characterized in that** the clamp assembly (5) is actuable for clamping to the bone by a relative longitudinal motion of the inner tube (3) and outer tube (4) with respect to each other.

3. Alignment apparatus according to claim 1 or 2, **characterized in that**
- the clamp assembly (5) comprises a second spreader (7, 8), the first and second spreaders (7, 8) being embodied with two rings (9, 10; 11, 12) distant from each other that are mounted on and movable along the inner tube (3), and which rings (9, 10; 11, 12) of any such spreader (7, 8) are connected to each other with flexible legs (13, 14);
- the first and second spreaders (7, 8) are provided adjacent to each other on the inner tube (3).

4. Alignment apparatus according to any one of claims 1 - 3, **characterized in that** the rings (10, 11) of the spreaders (7, 8) that are facing each other are provided with cooperating protrusions and recesses to provide that the spreaders (7, 8) can be rotationally fixed with respect to each other.

5. Alignment apparatus according to any one of claims 1 - 4, **characterized in that** the or each spreader (7, 8) is embodied with six legs (13, 14).

6. Alignment apparatus according to any one of claims 1 - 5, **characterized in that** at a proximal end (18) the apparatus is provided with a handle (19) and means (20) to move the inner tube (3) relative to the outer tube (4).

7. Alignment apparatus according to claim 6, **characterized in that** the means (2) to move the inner tube (3) relative to the outer tube (4) is embodied as a trigger (21) having at least one plate (22) which is provided with an aperture for passing through the inner tube (3), and that operating the trigger (21) initially causes the plate (22) to tilt with reference to the inner tube (3) and to provide a connection to the inner tube (3), such that with continued movement of the trigger (21) the inner tube (3) moves longitudinally within the outer tube (4) for actuating the clamp assembly (5).

8. Alignment apparatus according to claim 6 or 7, **characterized in that** at its proximal end (18) the apparatus (1) is provided with a release (23) for the inner tube (3), which release (23) is embodied with at least one plate (24) provided with an aperture for passing through the inner tube (3), and which plate (24) has a preferential position in which it is able to tilt relative to the inner tube (3) and to connect to said inner tube (3), wherein operating the release (23) will cause the plate (24) to be released and disconnected from the inner tube (3) so as to enable the inner tube (3) to move longitudinally within the outer tube (4) to a release position of the clamp assembly (5) which will bring it in disengagement with the intramedullary canal of the bone.

## Patentansprüche

1. Ausrichtvorrichtung (1) zum Führen eines Schneidelements entlang einer vorgegebenen Bahn in einem intramedullären Kanal eines menschlichen Knochens, wobei die Vorrichtung aufweist:
- einen Rahmen (2) zum Führen des Schneidelementes entlang der vorgegebenen Bahn;
- eine einstellbare Klemmbaugruppe (5), die mit dem Rahmen (2) verbunden ist, um den Rahmen (2) an den Knochen zu klemmen;
wobei:
- der Rahmen (2) zumindest eine Röhre (3, 4) mit einem Lumen zum Führen des Schneidelements aufweist und wobei der Rahmen (2) dazu eingerichtet ist, in den intramedullären Kanal eingeführt zu werden; und
- die Klemmbaugruppe (5) an einem distalen Ende (6) der zumindest einen Röhre (3, 4) vorgesehen ist;
- der Rahmen (2) eine Innenröhre (3) und eine Außenröhre (4) aufweist, die koaxial zueinander positioniert sind; wobei die Innenröhre (3) und die Außenröhre (4) dazu eingerichtet sind, in den intramedullären Kanal eingeführt zu werden;
- die Innenröhre (3) mit dem Lumen zum Führen des Schneidelements versehen ist;
- die Klemmbaugruppe (5) so konfiguriert ist, dass sie zum Festklemmen am Knochen durch eine Relativbewegung der Innenröhre (3) und der Außenröhre (4) in Bezug aufeinander betätigt werden kann;
**dadurch gekennzeichnet, dass**
- die Klemmbaugruppe (5) an der Innenröhre (3) vorgesehen ist und zumindest einen ersten Spreizer (7, 8) aufweist, der mit zwei voneinander beabstandeten Ringen (9, 10; 11, 12) ausgeführt ist, die an der Innenröhre (3) montiert und entlang dieser beweglich sind, und die mit flexiblen Schenkeln (13, 14) miteinander verbunden sind;
- ein erster Anschlag (15) an einem distalen Ende (6) der Innenröhre (3) vorgesehen ist, um zu verhindern, dass der erste Spreizer (7, 8) von der Innenröhre (3) abgleitet;
- ein zweiter Anschlag (16) durch ein distales Ende (17) der Außenröhre (4) gebildet ist, der die Bewegung von zumindest einem (12) der zwei Ringe des ersten Spreizers (7, 8) begrenzt;
- eine Relativbewegung der zwei Ringe (9, 10; 11, 12) des ersten Spreizers (7, 8) ein Biegen der flexiblen Schenkel (13, 14) bewirkt, so dass eine Annäherungsbewegung der zwei Ringe (9, 10; 11, 12) des ersten Spreizers (7, 8) die flexiblen Schenkel (13, 14) dazu veranlasst, sich nach außen zu biegen und in den intramedullären Kanal einzugreifen, um als eine Klammer innerhalb des intramedullären Kanals zu wirken.

2. Ausrichtvorrichtung gemäß Anspruch 1, **dadurch gekennzeichnet, dass** die Klemmbaugruppe (5) zum Klemmen an den Knochen durch eine Relativbewegung der Innenröhre (3) und der Außenröhre (4) zueinander in Längsrichtung betätigbar ist.

3. Ausrichtvorrichtung gemäß Anspruch 1 oder 2, **dadurch gekennzeichnet, dass**
- die Klemmbaugruppe (5) einen zweiten Spreizer (7, 8) aufweist, wobei der erste und der zweite Spreizer (7, 8) mit zwei voneinander beabstandeten Ringen (9, 10; 11, 12) ausgeführt sind, die an der Innenröhre (3) montiert und entlang dieser bewegbar sind, und wobei die Ringe (9, 10; 11, 12) eines jeden solchen Spreizers (7, 8) mit flexiblen Schenkeln (13, 14) miteinander verbunden sind;
- der erste und der zweite Spreizer (7, 8) nebeneinander auf der Innenröhre (3) vorgesehen sind.

4. Ausrichtvorrichtung gemäß einem der Ansprüche 1 - 3, **dadurch gekennzeichnet, dass** die einander zugewandten Ringe (10, 11) der Spreizer (7, 8) mit zusammenwirkenden Vorsprüngen und Ausnehmungen versehen sind, um zu gewährleisten, dass die Spreizer (7, 8) drehfest zueinander fixiert werden können.

5. Ausrichtvorrichtung gemäß einem der Ansprüche 1 - 4, **dadurch gekennzeichnet, dass** der oder jeder Spreizer (7, 8) mit sechs Schenkeln (13, 14) ausgeführt ist.

6. Ausrichtvorrichtung gemäß einem der Ansprüche 1 bis 5, **dadurch gekennzeichnet, dass** die Vorrichtung an einem proximalen Ende (18) mit einem Griff (19) und einem Mittel (20) zum Bewegen der Innenröhre (3) relativ zu der Außenröhre (4) versehen ist.

7. Ausrichtvorrichtung gemäß Anspruch 6, **dadurch gekennzeichnet, dass** das Mittel (2) zum Bewegen der Innenröhre (3) relativ zu der Außenröhre (4) als Auslöser (21) ausgebildet ist, der zumindest eine Platte (22) aufweist, die mit einer Öffnung zum Durchführen der Innenröhre (3) versehen ist, und dass ein Betätigen des Auslösers (21) zunächst bewirkt, dass die Platte (22) in Bezug auf die Innenröhre (3) kippt und eine Verbindung mit der Innenröhre (3) herstellt, so dass sich die Innenröhre (3) bei fortgesetzter Bewegung des Auslösers (21) in Längsrichtung innerhalb der Außenröhre (4) zum Betätigen der Klemmbaugruppe (5) bewegt.

8. Ausrichtvorrichtung gemäß Anspruch 6 oder 7, **dadurch gekennzeichnet, dass** die Vorrichtung (1) an ihrem proximalen Ende (18) mit einer Freigabeeinrichtung (23) für die Innenröhre (3) versehen ist, wobei die Freigabeeinrichtung (23) mit zumindest einer Platte (24) ausgeführt ist, die mit einer Öffnung zum Durchführen der Innenröhre (3) versehen ist, und wobei die Platte (24) eine Vorzugsposition besitzt, in der sie relativ zu der Innenröhre (3) kippen und sich mit der Innenröhre (3) verbinden kann, wobei das Betätigen der Freigabeeinrichtung (23) bewirkt, dass die Platte (24) freigegeben und von der Innenröhre (3) getrennt wird, um es der Innenröhre (3) ermöglichen, sich in Längsrichtung innerhalb der Außenröhre (4) zu einer Freigabeposition der Klemmbaugruppe (5) bewegen, die sie in eine Loslösung von dem intramedullären Kanal des Knochens bringt.

## Revendications

1. Appareil d'alignement (1) pour guider un élément de coupe le long d'une trajectoire prédéterminée dans un canal intramédullaire d'un os humain, ledit appareil comprenant :
- un bâti (2) pour guider l'élément de coupe le long de la trajectoire prédéterminée ;
- un ensemble formant dispositif de serrage ajustable (5) raccordé audit bâti (2) pour serrer le bâti (2) sur l'os ; dans lequel :
- le bâti (2) comprend au moins un tube (3, 4) avec une lumière pour guider l'élément de coupe, et lequel bâti (2) est agencé pour être inséré dans ledit canal médullaire ; et
- l'ensemble formant dispositif de serrage (5) est prévu au niveau d'une extrémité distale (6) de l'au moins un tube (3, 4) ;
- le bâti (2) comprend un tube interne (3) et un tube externe (4) qui sont positionnés de manière coaxiale l'un par rapport à l'autre ; lesquels tube interne (3) et tube externe (4) sont agencés pour être insérés dans ledit canal intramédullaire ;
- le tube interne (3) est muni de la lumière pour guider l'élément de coupe ;
- l'ensemble formant dispositif de serrage (5) est configuré pour pouvoir être actionné afin de se serrer sur l'os par un mouvement relatif du tube interne (3) et du tube externe (4) l'un par rapport à l'autre ;
**caractérisé en ce que** :
- l'ensemble formant dispositif de serrage (5) est prévu sur le tube interne (3) et comprend au moins un premier écarteur (7, 8) qui est mis en œuvre avec deux bagues (9, 10 ; 11, 12) à distance l'une de l'autre qui sont montées sur et mobiles le long du tube interne (3), et qui sont raccordées entre elles avec des pattes flexibles (13, 14) ;
- une première butée (15) est prévue au niveau d'une extrémité distale (6) du tube interne (3) pour empêcher que le premier écarteur (7, 8) sorte, par coulissement, du tube interne (3) ;
- une seconde butée (16) est mise en œuvre par une extrémité distale (17) du tube externe (4) qui limite le mouvement d'au moins l'une (12) des deux bagues du premier écarteur (7, 8) ;
- le mouvement relatif des deux bagues (9, 10 ; 11, 12) du premier écarteur (7, 8) provoque la flexion des pattes flexibles (13, 14), de sorte qu'un mouvement d'approche des deux bagues (9, 10 ; 11, 12) du premier écarteur (7, 8) provoque la flexion des pattes flexibles (13, 14) et la mise en prise du canal intramédullaire afin de servir de dispositif de serrage à l'intérieur dudit canal intramédullaire.

2. Appareil d'alignement selon la revendication 1, **caractérisé en ce que** l'ensemble formant dispositif de serrage (5) peut être actionné pour se serrer sur l'os par un mouvement longitudinal relatif du tube interne (3) et du tube externe (4) l'un par rapport à l'autre.

3. Appareil d'alignement selon la revendication 1 ou 2, **caractérisé en ce que** :
- l'ensemble formant dispositif de serrage (5) comprend un second écarteur (7, 8), les premier et second écarteurs (7, 8) étant mis en œuvre avec deux bagues (9, 10 ; 11, 12) à distance l'une de l'autre, qui sont montées sur et mobiles le long du tube interne (3), et lesquelles bagues (9, 10 ; 11, 12) de l'un quelconque de ces écarteurs (7, 8) sont raccordées entre elles avec des pattes flexibles (13, 14) ;
- les premier et second écarteurs (7, 8) sont prévus de manière adjacente entre eux sur le tube interne (3).

4. Appareil d'alignement selon l'une quelconque des revendications 1 à 3, **caractérisé en ce que** les bagues (10, 11) des écarteurs (7, 8) qui se font face, sont prévues avec des saillies et des évidements coopérants pour prévoir que les écarteurs (7, 8) puissent être fixes en rotation, l'un par rapport à l'autre.

5. Appareil d'alignement selon l'une quelconque des revendications 1 à 4, **caractérisé en ce que** le ou chaque écarteur (7, 8) est mis en œuvre avec six pattes (13, 14).

6. Appareil d'alignement selon l'une quelconque des revendications 1 à 5, l'appareil étant **caractérisé en ce que**, au niveau d'une extrémité proximale (18), il est muni d'une poignée (19) et d'un moyen (20) pour déplacer le tube interne (3) par rapport au tube externe (4).

7. Appareil d'alignement selon la revendication 6, **caractérisé en ce que** le moyen (2) pour déplacer le tube interne (3) par rapport au tube externe (4) est mis en œuvre sous la forme d'une gâchette (21) ayant au moins une plaque (22) qui est munie d'une ouverture pour passer à travers le tube interne (3), et **en ce que** l'actionnement de la gâchette (21) amène, initialement, la plaque (22) à s'incliner en référence au tube interne (3) et à fournir un raccordement avec le tube interne (3), de sorte qu'avec le mouvement continu de la gâchette (21), le tube interne (3) se déplace longitudinalement dans le tube externe (4) afin d'actionner l'ensemble formant dispositif de serrage (5).

8. Appareil d'alignement selon la revendication 6 ou 7, l'appareil (1) étant **caractérisé en ce que**, au niveau de son extrémité proximale (18), il est muni d'une libération (23) pour le tube interne (3), laquelle libération (23) est mise en œuvre avec au moins une plaque (24) munie d'une ouverture pour passer à travers le tube interne (3), et laquelle plaque (24) a une position préférentielle dans laquelle elle peut s'incliner par rapport au tube interne (3) et se raccorder audit tube interne (3), l'actionnement de la libération (23) amenant la plaque (24) à être libérée et déconnectée du tube interne (3) afin de permettre au tube interne (3) de se déplacer longitudinalement à l'intérieur du tube externe (4) jusqu'à une position de libération de l'ensemble formant dispositif de serrage (5) qui l'amène à se dégager du canal intramédullaire de l'os.
